# EUROPEAN PATENT APPLICATION

(11) **EP 3 496 109 A1**
(43) Date of publication of application: **12.06.2019**
(21) Application number: 17206225.9
(22) Date of filing: 08.12.2017
(51) Int. Cl.: G16H 50/20, G16H 30/40, A61B 5/085

(54) **OSCILLATORY DARK-FIELD IMAGING**

(71) Applicant: Koninklijke Philips N.V., 5656 AE Eindhoven (NL)
(72) Inventor: SABCZYNSKI, Jörg, 5656 AE Eindhoven (NL); KOEHLER, Thomas, 5656 AE Eindhoven (NL)
(74) Representative: de Haan, Poul Erik

(57) **Abstract**

A forced oscillation technique and dark field imaging technique is disclosed, wherein respiratory mechanics data and dark field image data are synergistically combined to obtain pulmonary function data with increased spatial resolution and increased diagnostic information, particularly increased localization and severity data.

## Description

### FIELD OF THE INVENTION

The present invention generally relates to a device, method and computer program for determining pulmonary function of a subject.

### BACKGROUND OF THE INVENTION

Lung diseases such as Chronic Obstructive Pulmonary Disease (COPD) and asthma affect a large part of the human population. COPD is a disease characterized by airflow obstruction. It affects about 330 million people, i.e. nearly 5% of the global population. Asthma is an inflammatory disease of the airways of the lungs. It affects about 300 million people worldwide. Early diagnosis and treatment prevents further deterioration of these diseases and improves the chance of curing or at least reducing the effects the disease, thereby increasing the wellbeing of the patient, as well as reducing medical costs.

Gas exchange in lungs takes place in membranes of the alveoli. The alveoli are small balloon-like tissue structures, which inflate and deflate during breathing. During inhalation, the alveoli are inflated, which requires an excess pressure in the inside relative to the surrounding tissue. During exhalation, the alveoli are deflated, either because of their elastic properties and alveolar wall tension or because of excess pressure on the outside of the alveoli due to diaphragm motion. During inhalation the radius of the alveoli increases from about 0.05 mm to 0.1 mm. This requires a pressure difference of about 1 mmHg in a healthy subject.
In lung diseases such as COPD and Asthma the alveoli do not inflate and/or deflate optimally, e.g. through damage to the alveoli themselves or through blockages or damages in other areas of the lung.

Lung diseases are usually assessed through pulmonary function tests in which the lungs, and particularly the alveoli, are activated or stimulated and the effect thereof is measured.

The most common pulmonary function test for lung diseases is spirometry, which is suitable to measure lung volume as well as airflow during inhalation and exhalation. During spirometry a subject needs to perform several respiratory maneuvers in order to generate the flow and volume changes that are measured. The results are then used in a diagnosis by a physician. A drawback of spirometry is that accuracy and reproducibility is limited due to strong dependence on capabilities, behavior and cooperation of the subject during the measurement, which makes the result rather inaccurate. Also, with spirometry it is only possible to measure integrated information on the respiration (volumes and flow) without spatial resolution.

Forced Oscillatory Technique (FOT) is an established alternative spirometry method to assess respiratory mechanics that does not need active subject cooperation and measures ventilation parameters during the physiological conditions of spontaneous breathing. During FOT low-amplitude low-frequency pressure oscillations are produced during spontaneous breathing without the need for difficult respiratory manoeuvers. By using this external excitation it is possible to measure the driving oscillatory pressure as well as the resulting oscillatory flow.

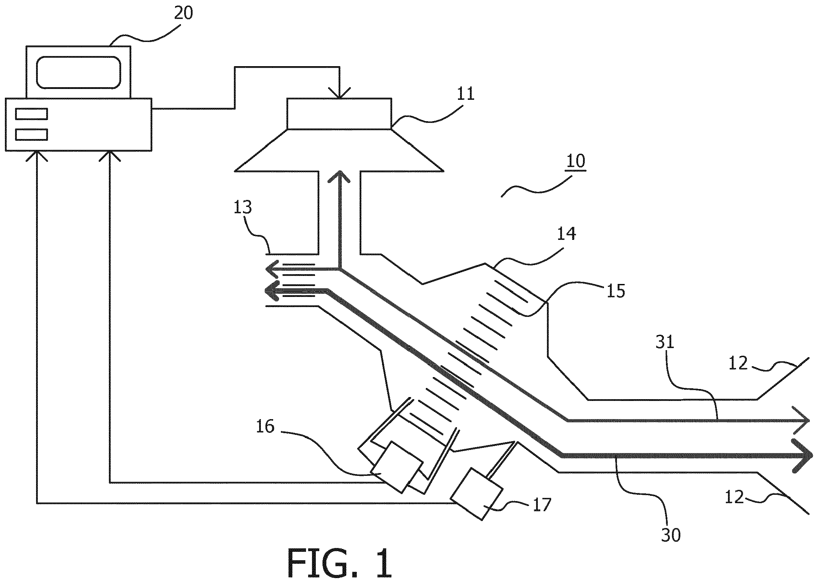

In a typical FOT setup 10 (as is sketched in Fig. 1), the subject breathes through a mouth piece 12 and a terminal resistor 13. The oscillations are induced using a loudspeaker 11 that is in parallel with the terminal resistor 13 and in open connection to the mouthpiece 12. The frequency and amplitude of the oscillations are transmitted from a control device 20. Due to the elastic properties of the lung tissue the air oscillations lead to an induced and controlled inflation and deflation of the alveoli. Flow is measured with a pneumotachograph, a technique using the Venturi effect in which air flow is led through an air path narrowing structure 15, such as a membrane, capillaries or lamellae, in a resistor 14. A differential pressure transducer 16 measures air pressure on both sides of the narrowing structure 15 and transmits the pressure difference to a computing device 20, in this example a computer 20 that acts both as computing device and control device. To reduce temperature dependency, the pneumotachograph may be equipped with a thermostat. Preferably mouth pressure is known to obtain more reliable data. This is measured with a further pressure transducer 17 in or near the mouth piece 12 and the measured mouth pressure is also transmitted to the computing device 20. The computing device 20 calculates and presents data indicative of the pulmonary function to a physician. Instead of a pneumotachograph, also other devices may be used to determine air flow, such as a turbine, ultrasound, hot wire anemometer or any other suitable device known to the skilled person. Also invasive alternatives may be used.

While FOT eliminates subject dependency, still it has only very limited spatial resolution and can only distinguish between upper airway obstruction and peripheral airway obstruction.

It would be advantageous to increase spatial resolution of pulmonary function tests such as spirometry and FOT.

### SUMMARY OF THE INVENTION

It is an object of the presently claimed invention to achieve the above and other advantages that will be clear to the skilled person.

Embodiments according to the present invention are directed to a device for determining a pulmonary function of a subject. The device comprises a forced oscillatory technique device that is configured to excite alveoli of a subject's lung with an oscillatory pressure to generate respiratory mechanics data of the subject. The device further includes a dark field x-ray imaging device that is configured to generate image data comprising dark field image data of the alveoli in at least a sub-section of the subject's lung in response to the oscillatory pressure.

Furthermore, the device comprises a computing device for generating pulmonary function data based on the respiratory mechanics data and the image data.

This device combines the advantages of FOT (patient-independent, integrated quantitative respiratory mechanics data) with those of DAX (increased spatial resolution). On top of that it is possible to better localize particular problem areas within the patient's lung and match the cause of the disease to the respiratory effect thereof.

Preferably, the device further comprises a controller that is configured to modulate an oscillatory frequency of the forced oscillatory technique device. The same or different controller is configured to modulate the image acquisition frequency of the dark field imaging device based on the oscillatory frequency device. Preferably the oscillatory frequency and the image acquisition frequency are in phase with each other.

Since the oscillatory frequency is known and the alveolic response may be derived from that, it is now possible to acquire DAX images at known alveolic states.

Preferably the dark field image data of the at least sub-section of the alveoli in maximum inflated state and dark field image data of the at least sub-section of the alveoli in maximum deflated state. As such the difference between maximum inflation and deflation may be analyzed, wherein a bigger difference indicates healthier alveoli and a smaller difference indicates potentially diseased alveoli (or hindered supply to the alveoli). This provides an additional measure of respiratory mechanics that may be cross-correlated with the FOT image to obtain improved and expanded information available for diagnosis.

Preferably, the dark field imaging device is further configured to acquire image data that comprises localization data comprising information on the position of the at least sub-section of the alveoli in the subject's lung, preferably localization information combined with information relating to the state of inflation or deflation of the alveoli. This allows for obtaining localized image data with respiratory mechanics information, which allows a physician to even more precisely diagnose a lung disease and to compare different sections of the patient's lung (e.g. healthy and diseased areas), for instance to obtain information on the severity of the condition.

Preferably, the controller is configured to modulate the oscillatory frequency with multiple different successive frequencies and/or to modulate the oscillatory frequency in a pulsed pattern. This allows for different types of oscillations to acquire FOT and DAX information on how the alveoli respond in different conditions (e.g. at high and low oscillatory frequencies).

Preferably, the dark field imaging device is a diagnostic x-ray device configured to generate 2D dark field image data or a diagnostic image device configured to generate 3D image data. These may be for instance diagnostic 2D x-ray devices, C-arm x-ray devices, tomosynthesis x-ray devices and computed tomography x-ray devices.

In one variation, the forced oscillation technique device is a non-invasive device arranged to directly receive a subject's exhalation. This allows for use of known FOT devices, including those based on pneumotachyograhpy.

In another variation, the forced oscillation technique device is an invasive device arranged to be inserted into a subject's airways. This allows for inducing oscillations in certain areas of the lung only and reduce DAX imaging to that area, reducing dose and allowing to better focus on a particular area of interest.

Preferably, the computing device is configured to generate a cross-correlation image based on a cross-correlation analysis performed between individual pixels, preferably every pixel, in the image data and a reference waveform over several oscillation cycles. The reference waveform is measured in a mouth piece of the forced oscillatory technique device or is generated by the computing device. This allows determining the time delay in phase of alveoli oscillation in different sections of the lung.

Another embodiment of the present invention is directed towards a corresponding method for determining a pulmonary function of a subject.

A further embodiment of the present invention is directed towards a computer program product configured to compute pulmonary function data based on respiratory mechanics data and image data as determined in said method.

Still further aspects and embodiments of the present invention will be appreciated by those of ordinary skill in the art upon reading and understanding the following detailed description. Numerous additional advantages and benefits will become apparent to those of ordinary skill in the art upon reading the following detailed description of preferred embodiments.

### BRIEF DESCRIPTION OF THE DRAWINGS

The present invention is illustrated by drawings of which
Fig. 1 shows a schematic illustration of a forced oscillation technique (FOT) device.
Fig. 2 shows a schematic illustration of a dark field x-ray (DAX) imaging device.
Fig. 3 shows a schematic illustration depicting imaging a part of a lung using a FOT-DAX device according to exemplary embodiments of the present invention.
Fig. 4 shows a schematic flow chart of a method to determine pulmonary function of a subject according to aspects of the present invention.
Fig. 5 shows diagrams of an exemplary induced oscillation pressure wave (a) and the alveolic response thereto and image acquisition moments (b).
Fig. 6 shows an exemplary schematic depiction of how pulmonary function data as obtained by FOT-DAX may be presented.

The invention may take form in various components and arrangements of components, and in various process operations and arrangements of process operations. The drawings are only for the purpose of illustrating preferred embodiments and are not to be construed as limiting the invention. To better visualize certain features may be omitted or dimensions may be not be according to scale.

### DETAILED DESRIPTION OF EMBODIMENTS

The present invention is based on the insight that superimposing pressure oscillations onto the air in the respiratory system (for instance by FOT) and to image the response of the lung tissue, especially of the alveoli, may be used synergistically to determine pulmonary function data that not only significantly reduces subject dependency, but also increases spatial resolution and provides precise information on the exact location in the lungs and severity of the impact of pulmonary diseases.

There is a great choice in imaging techniques, but most imaging techniques are unsuitable because they are too inaccurate, too slow and/or have insufficient contrast or spatial resolution. It is a further insight of the present invention that dark field x-ray imaging overcomes the above mentioned drawbacks of common imaging techniques, while it simultaneously delivers on increasing spatial resolution and localization lacking in FOT.

Dark field x-ray (DAX) imaging is an imaging technique that only recently found practical use in medical imaging. Dark field imaging is already long known for visual optics, but for x-ray imaging it was restricted to highly brilliant synchrotron x-ray sources that are not suitable for medical imaging due to their size and very limited energy band width and angular divergence. However, recently a grating-based solution was developed to generate dark field x-ray images using x-ray tubes commonly used in medical imaging [Pfeiffer et.al., "Hard-X-ray dark-field imaging using a grating interferometer", Nature Materials, Vol. 7, February 2008, page 134-137].

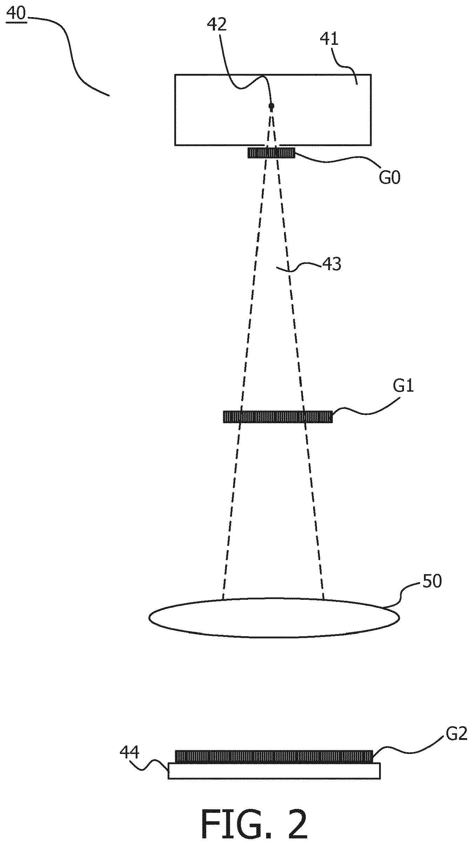

DAX may be performed using a DAX set-up 40 as is schematically sketched in Fig. 2. An X-ray beam 43 is emitted from an x-ray focal spot 42 of an x-ray source 41. A first grating structure G0, commonly named the source grating, is placed close to the focal spot 42. Because of the source grating G0 the radiation beam 43 is in effect divided into an array of line sources, which then pass through a second grating structure G1, commonly named the phase grating, which may be placed in front or behind a subject 50 to be imaged. The x-ray beam 43 passes a third grating structure G2, commonly named the analyzer grating, before it is detected by a detector 44, in which imaging information is generated and transmitted to processing equipment (not shown). Both the phase grating G1 and the analyzer grating G2 contribute to image contrast, wherein the phase grating G1 causes periodic spatial phase modulations in the x-ray beam 43. By propagation of the partially coherent x-rays, the phase modulation is transformed into an intensity modulation which has a typical period in the range of 5 - 50 µm (Talbot effect). The analyzer grating G2 then transforms the highfrequency intensity modulations into a low-frequency intensity modulation. When the phase grating G1 or the analyzer grating G2 is moved transversely with respect to the radiation beam 43, the x-ray intensity detected oscillates in each pixel of the detector 44, wherein the magnitude of the oscillations is determined by the subject 50. These local intensity changes may then be used to determine dark field image data. The obtained dark field image data represents scatter information of the x-ray beam 43 through the subject 50. This scatter data is obtained simultaneously with x-ray transmission image data, which provides attenuation measurement data, particularly of a difference between high and low absorption areas, and with phase contrast image data, which provides increased soft-tissue contrast.

This DAX set-up is very suitable, but the present invention would work with any DAX set-up suitable for medical imaging, e.g. variations with more or less gratings, crystals instead of gratings and any other variation known to a skilled person.

The dark field signal is particularly useful for strongly small-angle scattering soft tissue areas, such as the micro-structure of lung tissue. Unlike conventional x-ray imaging, where only attenuation (due to photo-electric absorption and Compton scattering) of the x-rays in a subject is measured, DAX imaging measures small angle scattering that occurs for instance at any air-tissue interfaces of the alveoli in the lung. In other words, the DAX signal is proportional to the number of these interfaces, which is proportional to the size of the alveoli.

While DAX itself provides information on the distribution and size of alveoli in a subject's lung, no functional information is generated, so therefore it would in itself be of limited use in lung diagnostics relating to respiratory diseases such as COPD and asthma.

By adding a FOT device 10 to the DAX set-up 40, the strengths of both diagnostic techniques remain, while they cancel out the weaknesses of each other and synergistically provide new type of pulmonary function data unobtainable by each individually.

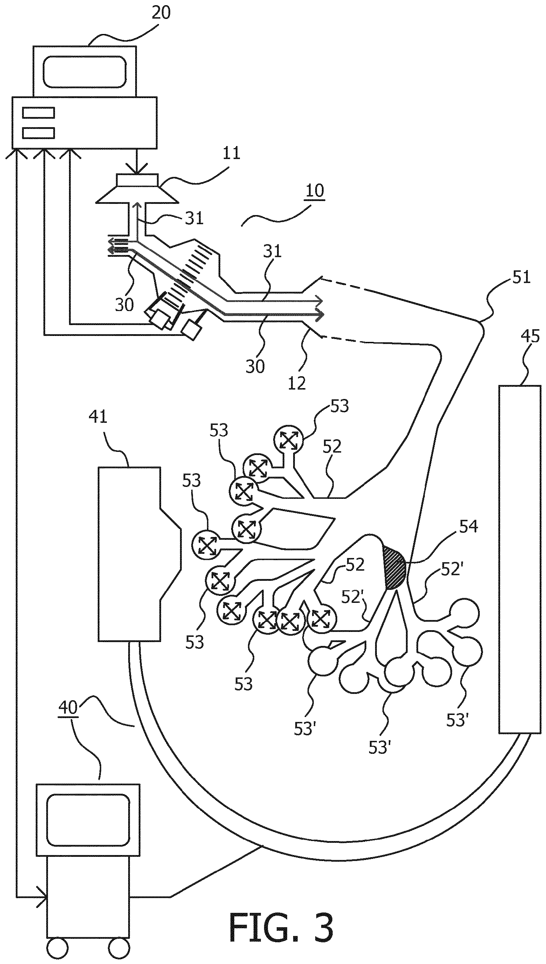

A schematic DAX-FOT set-up is shown in Fig. 3 comprising a controller 20, a DAX set-up 40 and an FOT device 10. Also shown is a (not to scale) section of the subject's lung comprising upper airways 51, bronchi 52, 52' and alveoli 53, 53' (for clarity reasons only part of the bronchi 52, 52' and alveoli 53, 53' are shown with reference numbers). In this example, the subject has a respiratory disease caused by obstructions 54, such as mucus, dust, pollution, tumors, etc., of some bronchi 52', as shown in Fig. 3. Due to said obstruction 54, air flow from the upper airways 51 to the alveoli 53' is severely obstructed. In other cases, the alveoli 53' might also function problematically due to other causes than obstructions, e.g. inflammation, infection, emphysema, cystic fibrosis or alveolic damage or porosity. The effect of these, potentially in combination with other symptoms, is similar in that the affected alveoli 53' do not inflate and deflate completely or even at all. In contrast, alveoli 53 of non-obstructed bronchi 52 do inflate and deflate properly (as is indicated by the arrows within the alveoli 53).

Using just FOT would result in detecting a reduced pulmonary function, but not the exact cause and location of the problem. Using just DAX would result in imaged alveoli 53, 53, but it is not known in which inflation or deflation state they are, making it not properly diagnose the pulmonary disease. Potentially a corresponding phase contrast image could reveal an obstruction or damage, but this is not linked to any functional pulmonary data. Performing FOT and then DAX, or vice versa, would not provide synergistic effects, since the respiratory state of the measurements are not matched and the FOT and DAX data cannot be properly correlated.

In the embodiment shown in Fig. 3 the subject 50 induces a respiratory flow 30 by breathing or blowing through the mouthpiece 12 of the FOT device 10. Since this is dependent on the condition, motivation and activity of the subject 50, this flow is usually not very well controlled, regular or reproducible. A computer 20 is used to control the FOT device 10 by transmitting the frequency and amplitude of forced oscillations generated in a loudspeaker 11 of the FOT device creating an oscillatory flow 31, which travels through the FOT device 10 and the upper airways 51 of the subject 41 towards the bronchi 52, 52' and the alveoli 53, 53'. When they are functioning properly the alveoli 53 inflate and deflate between a maximum inflation size and a maximum deflation size. The FOT device 10 provides pulmonary function data as described earlier, which is then transmitted to the computer 20, which in this embodiment is also configured to act as a computing device 20 to process and analyze data from the FOT device 20. While FOT is a preferred technique, other techniques that may cause alveoli 53, 53' to inflate and deflate in a controlled manner corresponding to regular breathing movement would be suitable as well.

During the FOT procedure the subject 50 and then particular a least part of the lungs of the subject 50, are imaged using a DAX imager 40. The DAX set-up 40 shown here is a C-arm diagnostic x-ray imager, but it could, for instance, also be another type of flat panel diagnostic x-ray imager or a computed tomography imager. The grating structure is not shown to avoid clutter in the image.

The subject's whole pulmonary area may be imaged, but it may also be a sub-section of the lungs to reduce radiation exposure of non-problematic areas and to reduce computational effort. This makes sense if it is already known where the problematic areas are. This may be known from a previous examination or from a low-dose scout scan performed before the DAX-FOT procedure.

During the procedure raw dark field image data is obtained through the DAX procedure, which in this embodiment is transmitted from the DAX device 40 to the computer 20, which is configured to also analyze dark field image data. The change in size of the alveoli during the procedure changes the contrast for DAX leading to different images for different states of inflation of the alveoli, thereby it is possible to determine the size and inflation state of the alveoli during the procedure.

During the FOT-DAX procedure transmission data and phase contrast image data is obtained simultaneously with the dark field image data, which may also be transmitted to the computer 20 and be analyzed and used in combination or separately from the dark field image data.

In this embodiment the computer 20 is also a control device configured to provide the DAX device with image acquisition data, such as image acquisition frequency that determines when a new dark field image is acquired. In the context of the present invention the term image acquisition frequency means the sequence in which a series of images is acquired with constant or varying time intervals between acquisitions of successive images.

In this embodiment the computer 20 controls the image acquisition frequency and phase such that it corresponds with acquiring images at moments when the alveoli are in their maximum inflated state and at moments where the alveoli are in their maximum deflated state. This may be achieved by acquiring a plurality of images during a period of oscillatory flow induced by the FOT device followed by analysis of the obtained dark field image data to select those in which the alveoli are in the maximum inflated and those in which the alveoli are in the maximum deflated stage. This is either a somewhat time consuming manual task or requires additional computational power in case of automatic selection. Also, the acquisition frequency may not be such that the alveoli are in their maximum inflated state and/or maximum deflated state. Because of this either many images must be taken to increase the chance that said maxima have been included on at least some of the DAX image data (and thereby increasing radiation dose and/or prolonging the procedure).

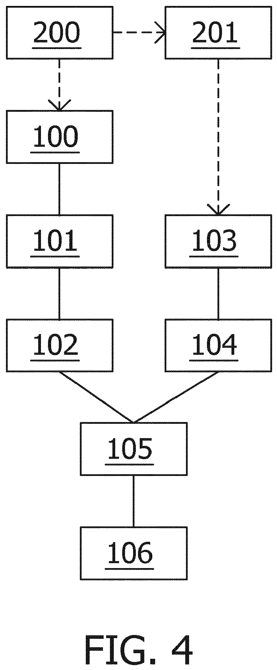

These issues may be solved by configuring the control device 20 for the FOT device and the DAX device such that it modulates the image acquisition frequency of the DAX device 40 based on the oscillatory frequency of the FOT device 10. Fig. 4 provides a simple flow diagram of this process. The induced oscillatory flow is induced 100 at a known frequency set 200 by the controlling device 20, therefore the pattern of inflating and deflating of alveoli is known. Measured respiratory mechanics data is acquired 101 from the oscillatory and respiratory flow data obtained from the FOT device 40 during the FOT-DAX procedure. The measured respiratory mechanics data is transmitted 102 to the computing device 20. The controlling device 20 sets 201 the image acquisition frequency and phase (e.g. such that they are in phase with each other) with the oscillation frequency such that raw DAX image data is acquired 103 at least the moments when the alveoli are in their maximum inflated state and at moments where the alveoli are in their maximum deflated state. Changing the phase difference between the induced FOT excitation and the imaging system acquisition results in a different contrast mechanism and different properties of the lung tissue are visible.

The oscillation frequency may be changed in order to cause different contrast. This allows also estimating the influence of the bronchi on the signal propagation to the alveoli and might be used to distinguish between for instance COPD and asthma.

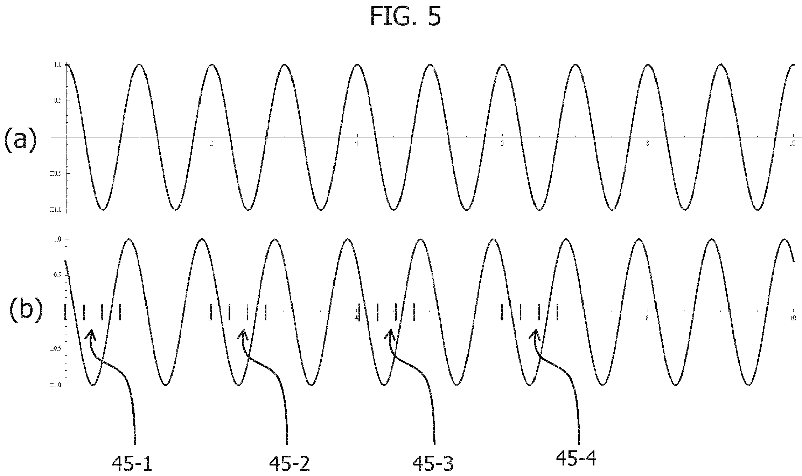

In an embodiment, the FOT device 40 is configured to generate sinusoidal pressure oscillations at a set frequency, typically in the range of 5 to 100 Hz. Assuming the lung responds linearly to the external pressure waves, it is desired to acquire DAX image data at least three different phases relative to the external pressure wave. This is illustrated in Fig. 5. Fig. 5a shows an example of an external excitation wave induced by the FOT device 40. Fig. 5b shows alveoli size in response to the external excitation. Both Fig. 5a and 5b have arbitrary units on the vertical axis and time (same scale for both) on the horizontal axis. The change in alveoli size has the same frequency as the induced oscillation frequency, but the alveoli response is in general somewhat phase shifted.

Phase retrieval is performed on images taken at the same relative phase to the external oscillation, i.e., from image *I*₁₁ to *I*₄₁ acquired with a first grating position for the first DAX image (45-1), *I*₁₂ to *I*₄₂ acquired with a second grating position for the second DAX image (45-2), et cetera (45-3, 45-4, ..). Using Fourier-analysis, derive maximum and minimum DAX signal levels may be derived from these individual images. In the exemplary diagram in Fig. 5b, four images are acquired within a single period of the excitation period (the moments of acquisition re indicated with vertical lines on the horizontal time axis). For high frequencies, these images may be distributed across different periods. In the example diagram of Fig. 5b, the images for subsequent grating positions are not acquired in subsequent periods of the excitation in order to allow for grating motion. This motion might also take longer than a single excitation period.

The raw dark field image data is transmitted 104 to the computing device 20. The computing device 20 then analyzes and combines the measured respiratory mechanics data and the raw dark field image data to generate 105 pulmonary function data that may be presented 106 to a user, such as a physician.

In the lung the driving pressure oscillation wave reaches different alveoli at different times depending on the length of the path in the bronchi and on the elasticity of the bronchi and bronchiole. In order to measure this time behavior the phase of the reference waveform may be tuned. This allows determining the phase of the alveoli oscillation. To achieve this, the computing device 20 may be configured to generate a cross-correlation image based on a cross-correlation analysis between individual pixels in the dark field image data and the reference waveform over several oscillation cycles. The reference waveform may be measured in the mouth piece 12 of the FOT device or may be generated by the control device 20. In an embodiment this cross-correlation image is performed for every pixel.

Areas of the lung with obstructions or with differing elastic parameters show different image contrast, since the oscillatory driving signal is differently damped before reaching the alveoli. Therefore the method may also be used to estimate the location and dimension of obstructions as well.

The generated pulmonary function data may comprise processed respiratory mechanics data and processed dark field image data and may be presented separately or in a combined or integrated manner or the user may select how and which data of the pulmonary function data is presented.

The pulmonary function data may be presented 106 visually on a display, e.g. the display of the computer 20 or it may be transmitted to another computer or workstation or a printer.

The computing device 20 may analyze the raw dark field image data to determine an inflation coefficient, defined as a difference in size (e.g. radius, diameter or volume) of an alveolus (or an average difference of a group of alveoli) between the maximum inflated state and the maximum deflated state. The computing device may then provide localization information of different areas with different inflation coefficients. The localization information may be included with the pulmonary function data and presented to the user on a displayed 2D or 3D dark field image indicating these different areas, for instance by highlighting areas with an inflation coefficient that is lower than a certain threshold (indicating reduced functioning of these alveoli) or providing a color coded map with different colors representing different inflation coefficients (e.g. green for larger values representing healthy alveoli and red for lower values representing potentially unhealthy alveoli).

The pulmonary function data may also additionally comprise phase contrast image data and/or transmission image data. Also further diagnostic support data (e.g. automated tumor detection or tracking) may be determined from all available data and presented to a user.

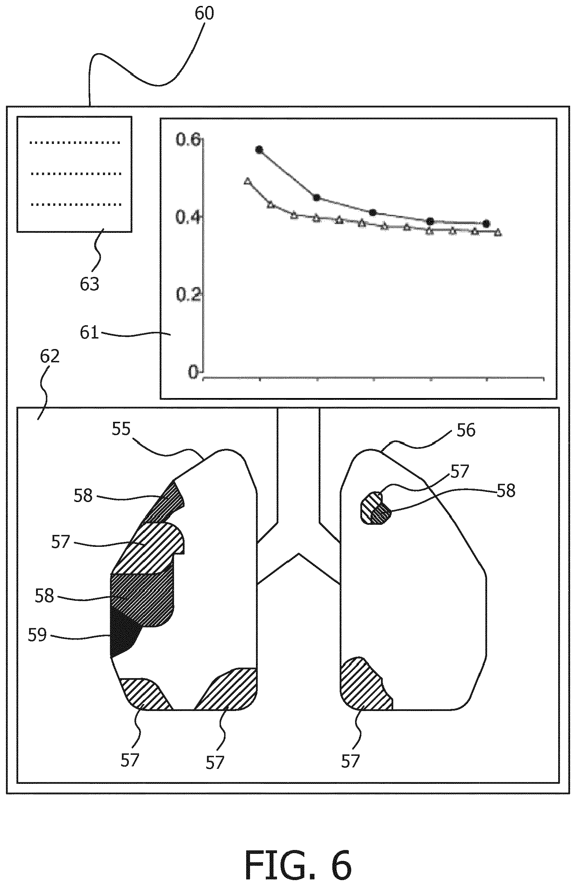

Fig. 6 shows a schematic exemplary FOT-DAX display image 60 of pulmonary function data obtained from a FOT-DAX procedure according to aspects of the present invention. This display image 60 comprises a processed respiratory mechanics data image 61 based on the FOT measurement; a processed DAX image 62 based on the raw DAX image data and meta data 63 regarding the conditions of the measurement and other relevant data to assist diagnosis.

The processed respiratory mechanics data image 61 corresponds with normal FOT data and presents an integrated view of the pulmonary function of a lung of a subject as a whole. Here the processed respiratory mechanics data image 61 is shown as a resistance graph, but it may also be presented differently or in combination with other commonly used data (e.g. a reactance graph, amplitude pressure graph, etc.). The processed respiratory mechanics data image 61 may also consist of or comprise textual or numerical data, such as measurement values. In other embodiments the user may select and/or toggle between different types of processed respiratory mechanics data image 61.

The processed DAX image 62 is shown here as a schematic drawing of a dark field image of the lung of the subject 50. The left lung lobe 56 and the right lung lobe 55 both show areas in which a white area indicates areas with a large inflation coefficient (healthy areas) and a dark area 59 indicates a low inflation coefficient (strongly unhealthy areas). The areas 57, 58 with differently spaced hatch patterns indicate areas with intermediate inflation coefficients, wherein a smaller spacing between hatch lines indicates a lower inflation coefficient (in other words: the closer the hatch lines are to each other, the more unhealthy the alveoli are in that area). The DAX image may also be zoomable.

The meta data 63 may comprise information on the oscillatory frequency, the acquisition frequency and other data related to the measurement and imaging conditions, as well as patient specific data, such as identification data, weight, age, (preliminary) diagnosis, responsible physician etc.

The data may in some embodiments also be manipulatable by the user through a user interface, e.g. setting thresholds for different inflation coefficient levels, toggling between dark field, phase contrast and transmission images.

Another option may be to compare the pulmonary function data with earlier obtained pulmonary function data to see if the subject's pulmonary health improved or deteriorated. The computational device 20 may automatically compare the current and earlier pulmonary function data and display only the difference or present both overlaid over each other.

Other forced oscillation techniques may also be used (as described previously), as long as they induce a controlled inflation and expansion of the alveoli. In one particular example, a driving pressure oscillation may be applied during invasive bronchoscopy, wherein an instrument (bronchoscope) is inserted into the airways, usually through the mouth or nose. The forced oscillations may be induced e.g. by a loudspeaker or other device at the tip of the bronchoscope. If necessary, the branch of interest of the bronchial tree can be sealed off with a balloon. As such it is possible to measure a single lung lobe, lung segment or branch. A drawback of invasive FOT-DAX is that the FOT device 10 may be imaged as well, obscuring parts of the lung or causing artifacts due to its presence.

Another variation may be to use a pulsed driving signal instead of a continuously oscillating driving signal. After each pulse images may then be acquired in order to measure temporal response of the tissue. The pulse is then repeated and the measurements are summed up, providing further information on the flexibility and recovery power of the lung tissue, including the alveoli.

Even though the main advantage of FOT compared to spirometry is that while it does not require a breath-hold, it will also work in a breath-hold mode. Since DAX imaging usually requires several x-ray projections taken of the same object, it is sensitive to motion, i.e. resulting in motion artefacts in the image data. Therefore, imaging during a breath-hold might be used to reduce these motion sensitivities. During breath-hold the patient must not close the trachea with the epiglottis. This requires either a cooperative patient or a method to detect this situation during imaging. A standard FOT setup allows automatically detecting a closed epiglottis since the resistance of the airway system is completely different than for an open epiglottis. This also allows triggering the DAX imaging.

While the invention has been illustrated and described in detail in the drawings and foregoing description, such illustration and description are to be considered illustrative or exemplary and not restrictive; the invention is not limited to the disclosed embodiments.

For instance the subject may be a human subject or an animal subject.

Further, in the described embodiments the computer 20 is a combined FOT-DAX control device 20 and computing device 20 and performs all control and computational tasks. Alternatively, individual control and/or computational tasks may be performed by separate devices as well, e.g. a computer connected to the FOT device and a computer dedicated (or integrated with) the DAX device 40. Also other or more dedicated computational devices and control devices may be separately available. The devices do not have to be in the same location and include remote devices, such as remote devices that provide cloud computation and the like. In such embodiments the different computing and control devices must be linked such that sufficient data band with is available for fast transfer of data between the different computing and control devices.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. Device for determining a pulmonary function of a subject (50) comprising
a forced oscillatory technique device (10) configured to excite alveoli (53, 53') of a subject's lung with an oscillatory pressure to generate respiratory mechanics data of the subject;
a dark field x-ray imaging device (40) configured to acquire image data comprising dark field image data of the alveoli in at least a sub-section of the subject's lung in response to the oscillatory pressure;
a computing device (20) for generating pulmonary function data based on the respiratory mechanics data and the image data.

2. Device according to claim 1, further comprising a controller (20) configured to modulate an oscillatory frequency of the forced oscillatory technique device (10) and the same or a different controller (20) configured to modulate the image acquisition frequency of the dark field imaging device (40) based on the oscillatory frequency device, preferably such that the oscillatory frequency and the image acquisition frequency are in phase with each other.

3. Device according to claim 1 or 2, wherein the dark field imaging device (40) is configured to acquire dark field image data of the at least sub-section of the alveoli in maximum inflated state and dark field image data of the at least sub-section of the alveoli in maximum deflated state.

4. Device according to any of the previous claims, wherein the dark field imaging device (40) is further configured to acquire image data that comprises localization data comprising information on the position of the at least sub-section of the alveoli (53, 53') in the subject's lung, preferably localization information combined with information relating to the state of inflation or deflation of the alveoli.

5. Device according to any of claims 2 to 4, wherein the controller (20) is configured to modulate the oscillatory frequency with multiple different successive frequencies and/or to modulate the oscillatory frequency in a pulsed pattern.

6. Device according to any of the previous claims, wherein the dark field imaging device (40) is a diagnostic x-ray device configured to generate 2D dark field image data or a diagnostic image device configured to generate 3D image data.

7. Device according to any of the previous claims, wherein the forced oscillation technique device (10) is a non-invasive device arranged to directly receive a subject's exhalation or an invasive device arranged to be inserted into a subject's airways.

8. Device according to any of the previous claims, wherein the computing device (20) is configured to generate a cross-correlation image based on a cross-correlation analysis between individual pixels, preferably every pixel, in the image data and a reference waveform over several oscillation cycles, wherein the reference waveform is measured in a mouth piece 12 of the forced oscillatory technique device (10) or is generated by the computing device.

9. Method for determining a pulmonary function of a subject 50 comprising the steps of
exciting (100) alveoli (53, 53') of the subject's lung with an applied oscillatory pressure at an oscillation frequency;
generating (101) respiratory mechanics data of the subject;
obtaining (103) image data image of at least a sub-section of the alveoli of the subject's lung in response to the oscillatory pressure;
computing (105) pulmonary function data based on the respiratory mechanics data and the image data; and
providing (106) the pulmonary function data to a user.

10. Method according to claim 9, wherein the image data comprises dark field image data.

11. Method according to claim 9 or 10, wherein the alveoli are excited (100) and respiratory mechanics data is generated (101) using a forced oscillation technique device (10).

12. Method according to any of claims 9 to 11, wherein the image data comprises image data of the at least sub-section of the alveoli in maximum inflated state and in maximum deflated state and/or localization data comprising information on the position of the at least sub-section of the alveoli (53, 53') in the subject's lung.

13. Method according to any of claims 9 to 12, wherein the image data was obtained at an image acquisition frequency that is based on the oscillatory frequency of the oscillation pressure, preferably the oscillatory frequency and the image acquisition frequency are in phase with each other.

14. Method according to any of claims 9 to 13, wherein a cross-correlation image is generated based on a cross-correlation analysis between individual pixels, preferably every pixel, in the image data and a reference waveform over several oscillation cycles, wherein the reference waveform is measured in a mouth piece 12 of the forced oscillatory technique device (10) or is generated by the computing device.

15. Computer program product configured to compute pulmonary function data based on respiratory mechanics data and image data as determined in the method of any of the claims 9 to 14.
